(19) Europäisches Patentamt
European Patent Office
Office européen des brevets

(11) Veröffentlichungsnummer: **0 001 113**
**B1**

(12) **EUROPÄISCHE PATENTSCHRIFT**

(45) Veröffentlichungstag der Patentschrift: **11.11.81**

(21) Anmeldenummer: **78100832.1**

(22) Anmeldetag: **06.09.78**

(51) Int. Cl.³: **C 07 D 513/04,**
A 61 K 31/54 //C07D333/34,
C07D333/38, (C07D513/04,
333/00, 279/00)

# (54) Thienothiazinderivate, deren Herstellung, sowie diese enthaltende Präparate.

(30) Priorität: **06.09.77 LU 78083**
**21.07.78 CH 7908/78**

(43) Veröffentlichungstag der Anmeldung:
**21.03.79 Patentblatt 79/6**

(45) Bekanntmachung des Hinweises auf die Patenterteilung:
**11.11.81 Patentblatt 81/45**

(84) Benannte Vertragsstaaten:
**BE CH DE FR LU NL SE**

(56) Entgegenhaltungen:
**FR - A - 2 282 893**

(73) Patentinhaber: **F. HOFFMANN-LA ROCHE & CO.**
**Aktiengesellschaft**
**CH-4002 Basel (CH)**

(72) Erfinder: **Pfister, Rudolf, Dr.**
**Neubadstrasse 128**
**4054 Basel (CH)**
Erfinder: **Zeller, Paul, Dr.**
**Rosenbergweg 52**
**4122 Allschwil (CH)**
Erfinder: **Binder, Dieter, Prof. Dr.**
**Blümelgasse 1**
**Wien 6 (AT)**
Erfinder: **Hromatka, Otto, Prof. Dr.**
**Starkfriedgasse 89**
**Wien 19 (AT)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 113 B1

Thienothiazinderivate, deren Herstellung, sowie diese enthaltende Präparate

Die Erfindung betrifft Thienothiazinderivate der allgemeinen Formel I

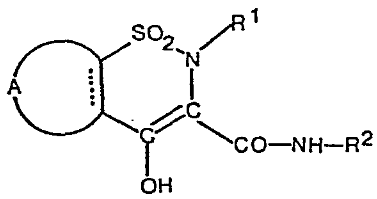

(I)

worin A mit den beiden Kohlenstoffatomen des Thiazinringes die Gruppe

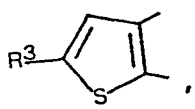

,

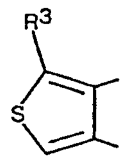

oder

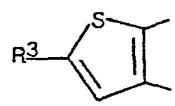

bildet und die gestrichelte Linie die im ersten und letzten Fall vorliegende Doppelbindung anzeigt, $R^1$ niederes Alkyl bedeutet, $R^2$ 2-Thiazolyl, 4-Methyl-2-thiazolyl, 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl oder einen gegebenenfalls durch Halogen, Hydroxy, niederes Alkyl, Trifluormethyl oder niederes Alkoxy substituierten Phenylrest bedeutet, und $R^3$ Halogen bedeutet,
sowie Salze davon.

Der in dieser Beschreibung verwendete Ausdruck "niederes Alkyl" bezeichnet geradkettige oder verzweigte gesättigte Kohlenwasserstoffgruppen mit 1-4 Kohlenstoffatomen, wie z.B. Methyl, Aethyl, Propyl, Isopropyl und t-Butyl. Der Ausdruck "niederes Alkoxy" bezieht sich auf Hydrocarbonoxygruppen mit bis zu 4 C-Atomen. Die Bezeichnung "Halogen" bezieht sich auf die 4 Halogene Chlor, Brom, Fluor, Jod.

In einer bevorzugten Klasse von Verbindungen der Formel I bedeutet $R^3$ Chlor oder Brom, wobei Chlor besonders bevorzugt ist. $R^1$ bedeutet vorzugsweise die Methylgruppe. $R^2$ stellt vorzugsweise 2-Thiazolyl, 5-Isoxazolyl oder 2-Pyridyl dar. A ist vorzugsweise die Gruppe

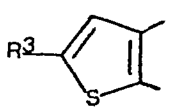

oder

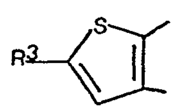

Besonders bevorzugte Verbindungen sind 6 - Chlor - 4 - hydroxy - 2 - methyl - 3 - (2 - pyridyl - carbamoyl) - 2H - thieno[2,3 - e]1,2 - thiazin - 1,1 - dioxid und das 6 - Chlor - 4 - hydroxy - 2 - methyl - 3 - (2 - pyridyl - carbamoyl) - 2H - thieno[3,2 - e]1,2 - thiazin - 1,1 - dioxid.

Die Thienothiazinderivate der allgemeinen Formel I werden erfindungsgemäss dadurch hergestellt, dass man

a) eine Verbindung der allgemeinen Formel II

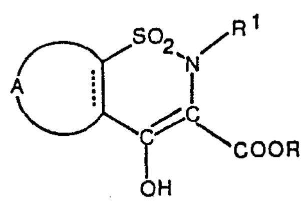

II

worin R niederes Alkyl bedeutet und A und $R^1$ die genannte Bedeutung haben, mit einem Amin der allgemeinen Formel III

$$H_2N—R^2 \qquad (III)$$

worin $R^2$ die genannte Bedeutung hat, umsetzt, oder

b) ein reaktionsfähiges funktionelles Derivat einer Säure der allgemeinen Formel IV

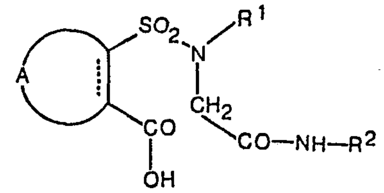

IV

worin A, $R^1$ und $R^2$ die genannte Bedeutung haben, cyclisiert, oder dass man

c) eine Verbindung der allgemeinen Formel V

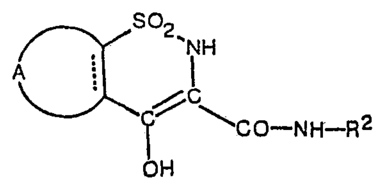

V

worin A und $R^2$ die genannte Bedeutung haben, mit einem den Rest $R^1$ liefernden Mittel alkyliert, oder

d) eine Verbindung der allgemeinen Formel VI

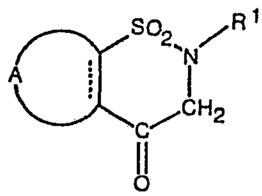

VI

worin A und $R^1$ die genannte Bedeutung haben, in Gegenwart einer starken Base mit einem Isocyanat der allgemeinen Formel VII

$$O{=}C{=}N{-}R^2 \qquad (VII)$$

worin $R^2$ die genannte Bedeutung hat, umsetzt, oder

e) ein Enamin der allgemeinen Formel VIII

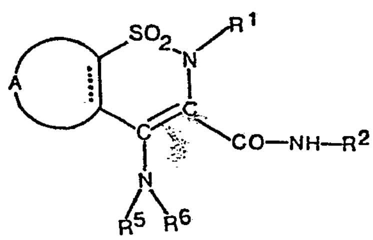

VIII

worin A, $R^1$ und $R^2$ die genannte Bedeutung haben, und $R^5$ und $R^6$ je nieder Alkyl bedeuten oder gemeinsam mit dem Stickstoffatom einer Pyrrolino-, Pyrrolidino-, Piperidino-, Morpholino- oder N-(nieder Alkyl)-piperazino rest bilden,
hydrolysiert, und

f) erwünschtenfalls eine so erhaltene Verbindung der allgemeinen Formel I in ein pharmazeutisch annehmbares Salz überführt.

Die Umsetzung nach Verfahrensaspekt a) kann in Anoder Abwesenheit eines inerten Lösungsmittels erfolgen. Als Lösungsmittel eignen sich Alkohole, wie Aethanol, Kohlenwasserstoffe, wie Benzol, Toluol, Xylol, halogenierte Kohlenwasserstoffe, wie Chloroform, Chlorbenzol, Methylenchlorid, Tetrachlorkohlenstoff, oder Dimethylformamid oder Dioxan. Die Umsetzung erfolgt vorzugsweise durch Erwärmen, wobei Schmelz- bzw. Rückflusstemperatur des Reaktionsgemisches besonders bevorzugt ist.

Nach dem Verfahrensaspekt b) gemäss Erfindung wird ein reaktionsfähiges funktionelles Derivat einer Verbindung der allgemeinen Formel IV cyclisiert. Diese Cyclisation erfolgt in Gegenwart einer Base und vorzugsweise in Gegenwart eines Lösungsmittels bei einer Temperatur zwischen 0°C und Rückflusstemperatur des Reaktionsgemisches, vorzugsweise zwischen Raumtemperatur und 60°C. Als Basen kommen insbesondere Hydride, Amide oder Alkoholate von Alkalimetallen in Betracht. Als Lösungsmittel eignen sich aprotische und protische, wie Alkohole, z.B. Methanol, Aethanol, Aether, z.B. Dioxan und Säureamide, z.B. Dimethylformamid. Zweckmässigerweise wird die Cyclisation so durchgeführt, dass man die Ausgangsverbindung der allgemeinen Formel IV im Lösungsmittel löst, mit der Base versetzt und das Reaktionsgemisch während 1 bis 4 Stunden entweder bei Raumtemperatur stehen lässt oder auf eine Temperatur bis zu 60°C erwärmt. Als reaktionsfähige funktionelle Derivate der Verbindungen der allgemeinen Formel IV eignen sich insbesondere deren niedere Alkylester, z.B. deren Methylester.

Nach dem Verfahrensaspekt c) wird eine Verbindung der allgemeinen Formel V alkyliert. Diese Alkylierung wird zweckmässigerweise so durchgeführt, dass man die Ausgangsverbindung der allgemeinen Formel V in einem aprotischen Lösungsmittel, wie Acetonitril, Dioxan oder Dimethylformamid, löst, mit einem Alkalimetallamid oder -hydrid in das Alkalimetallsalz überführt und dann durch Behandeln mit einem Alkylierungsmittel, insbesondere einem Alkylhalogenid oder -sulfat, in die entsprechende Verbindung der allgemeinen Formel I überführt. Temperatur und Druck sind für dieses Verfahren nicht kritisch, so dass die Reaktion der Einfachheit halber vorzugsweise bei Raumtemperatur und Atmosphärendruck durchgeführt wird.

Nach dem Verfahrensaspekt d) wird eine Verbindung der allgemeinen Formel VI in Gegenwart einer starken Base mit einem Isocyanat der allgemeinen Formel VII umgesetzt. Als starke Basen eignen sich Alkaliamide, Alkali- oder Erdalkalihydride sowie metallisches Alkali oder Erdalkali. Die Umsetzung erfolgt vorzugsweise unter einem Inertgas, z.B. Stickstoff, bei einer Temperatur zwischen 0—50°C, vorzugsweise bei Raumtemperatur, und in Gegenwart eines inerten, polaren Lösungsmittels, wie Toluol, Dioxan, Dimethylformamid, Dimethylsulfoxyd oder Hexamethylphosphorsäuretriamid (HMTP). Die als Ausgangsstoffe benötigte Isocyanate der allgemeinen Formel VII sind entweder bekannt oder können in Analogie zur Herstellung der bekannten Vertreter synthetisiert werden.

Nach dem Verfahrensaspekt e) wird ein Enamin der allgemeinen Formel VIII hydrolysiert. Die Hydrolyse erfolgt vorzugsweise mit einer wässrigen Mineralsäure, wie Chlorwasserstoffsäure, Bromwasserstoffsäure, Jodwasserstoffsäure, Schwefelsäure, oder mit Trifluoressigsäure, wobei bei einer Temperatur zwischen 50°C und dem Siedepunkt des Reaktionsgemisches, vorzugsweise dem Siedepunkt des Reaktionsgemisches gearbeitet wird. Bei dieser Hydrolyse dient die Säure gleichzeitig als Lösungsmittel.

Die Ausgangsverbindungen für das Verfahren gemäss Variante a) können ausgehend von bekannten Produkten in an sich bekannter Weise hergestellt werden. Insbesondere können sie gemäss den folgenden Reaktionsschematas und gemäss den spezifischen Angaben in den Beispielen synthetisiert werden. Reaktionsschema I bezieht sich auf Verbindungen.

worin A 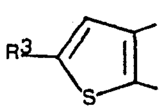 Reaktionsschema II auf solche,

worin A 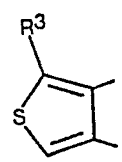 und Reaktionsschema III

auf solchen, worin A 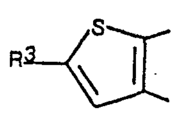 bedeutet.

Reaktionsschema I

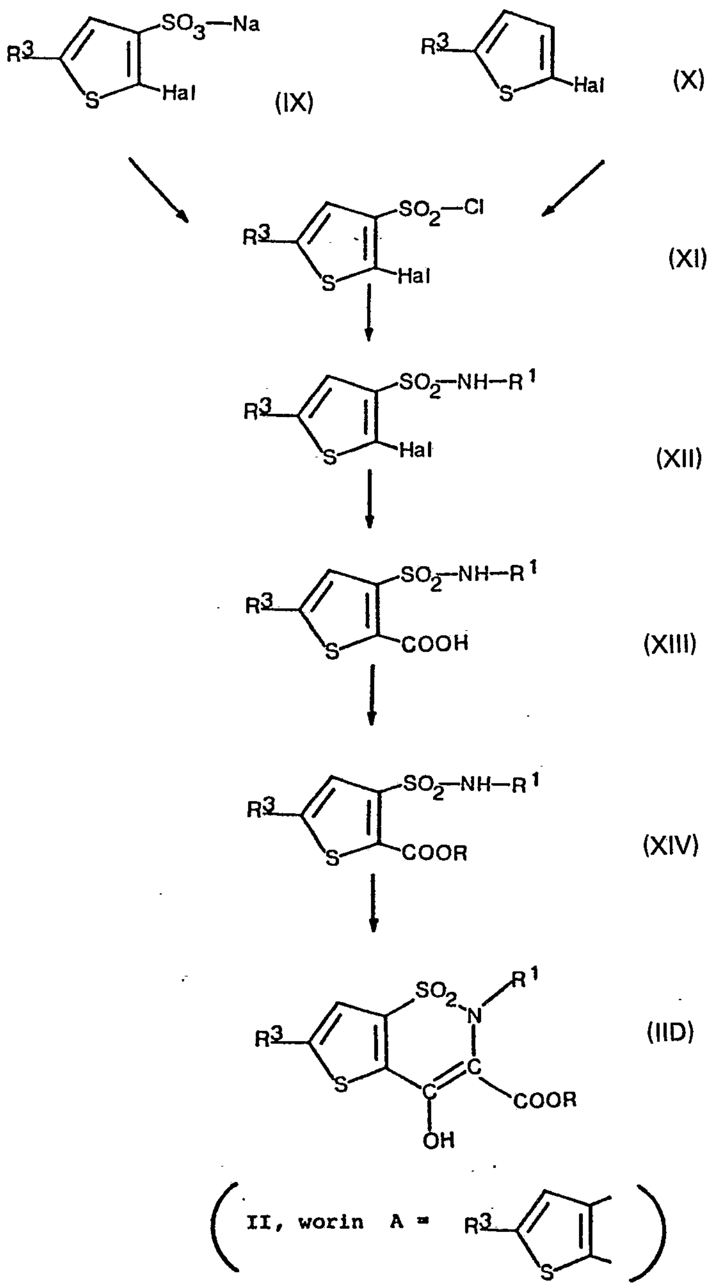

(IX) (X)

(XI)

(XII)

(XIII)

(XIV)

(IID)

(II, worin A =

Reaktionsschema II

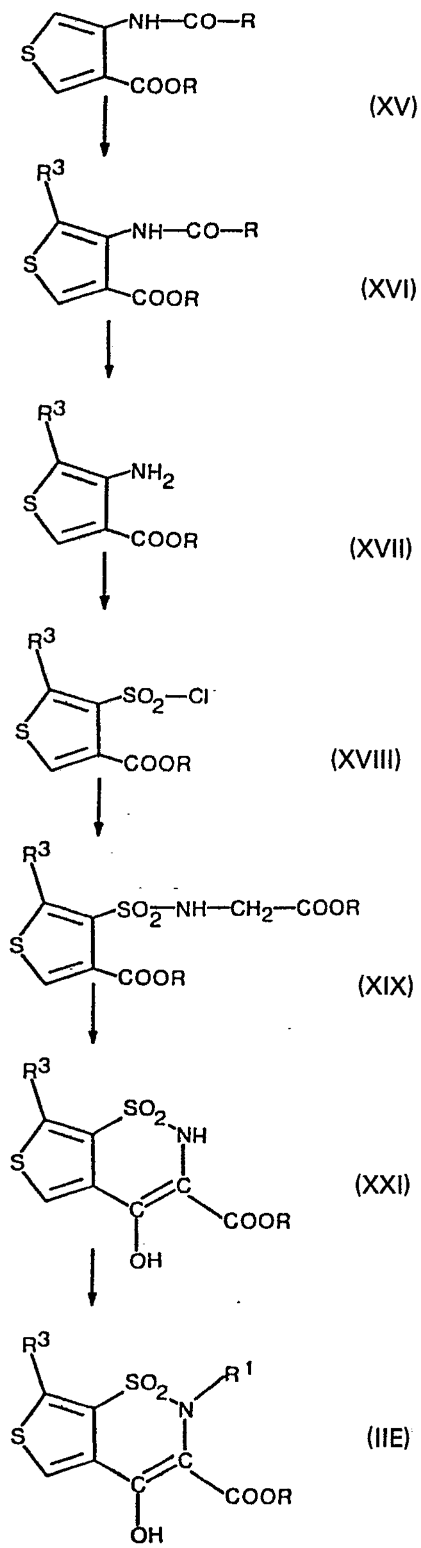

(XV)

(XVI)

(XVII)

(XVIII)

(XIX)

(XXI)

(IIE)

(II, worin A = 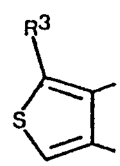 )

Reaktionsschema III

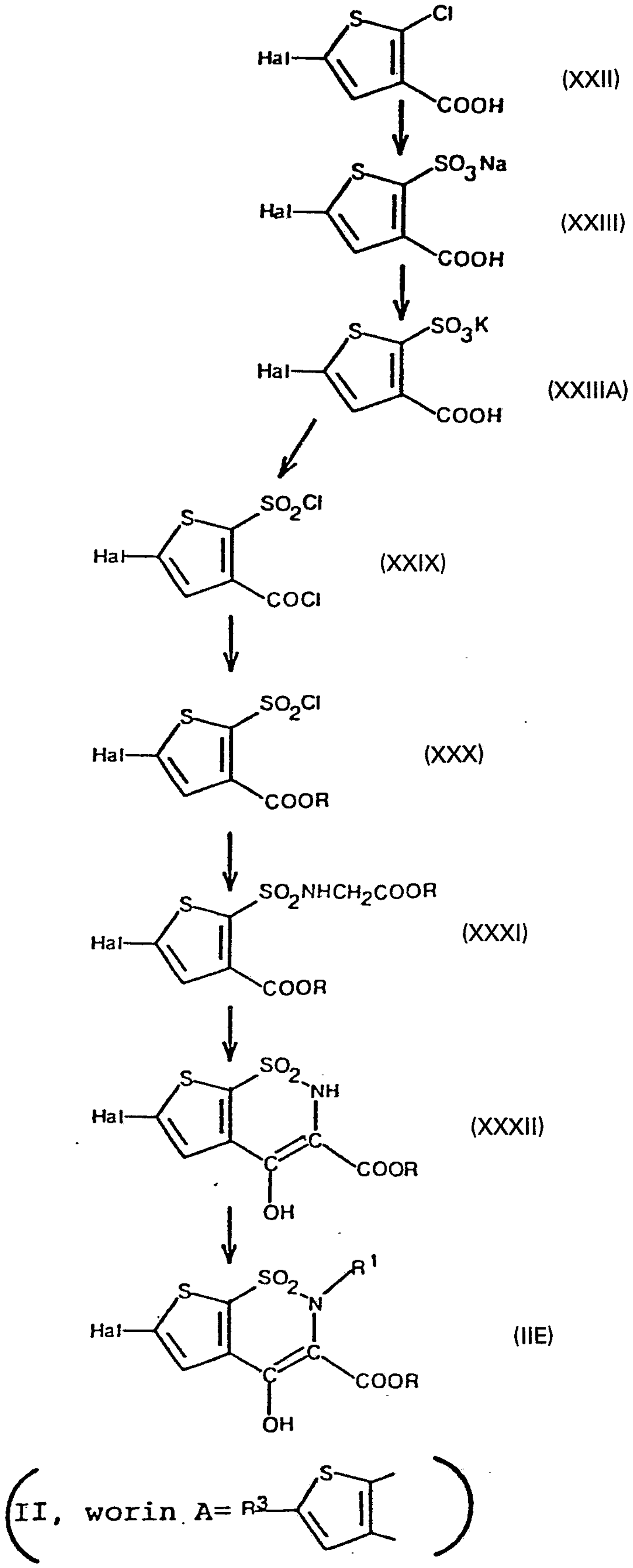
(XXII)
(XXIII)
(XXIIIA)
(XXIX)
(XXX)
(XXXI)
(XXXII)
(IIE)
(II, worin A= )

Für Verfahrensaspekt b) als Ausgangsverbindungen verwendbare niedere Alkylester von Säuren der allgemeinen Formel IV können z.B. erhalten werden, indem man eine Amin der allgemeinen Formel III mit Chloracetylchlorid umsetzt und das erhaltene Produkt der allgemeinen Formel

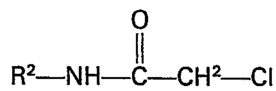

worin $R^2$ die genannte Bedeutung hat, mit einer Verbindung der allgemeinen Formel XIV umsetzt. Andere reaktionsfähige funktionelle Derivate von Säuren der allgemeinen Formel IV können in an sich bekannter Weise aus den so erhaltenen Estern hergestellt werden.

Für Verfahren c) verwendbare Ausgangsstoffe der allgemeinen Formel V können z.B. durch Umsetzen einer Verbindung der allgemeinen Formel XXI, XXVIII oder XXXII mit einem Amin der allgemeinen Formel III erhalten werden.

Die für Verfahren d) benötigten Ausgangsstoffe der allgemeinen Formel VI können nach an sich bekannten Methoden hergestellt werden.

Die Herstellung der Ausgangsstoffe für Verfahren e) kann nach dem folgenden Reaktionsschema IV erfolgen.

Reaktionsschema IV

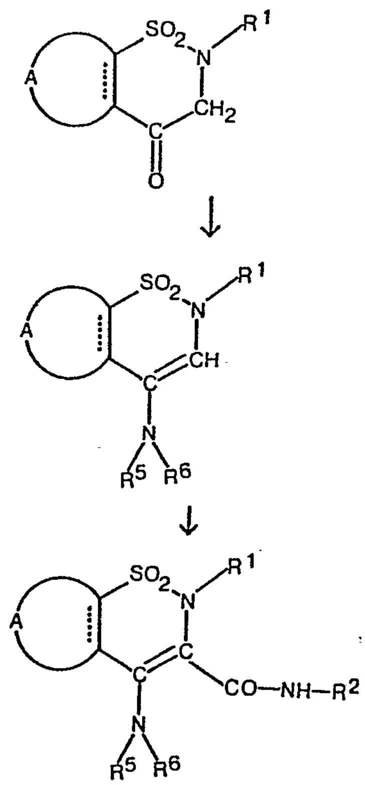

(VIII)

Die Verbindungen der allgemeinen Formel I sind sauer und können mit entsprechenden Basen pharmazeutisch annehmbare Salze bilden. Als Basen eignen sich z.B. Alkalimetalle, wie Lithium, Natrium, Kalium; Erdalkalien, wie Magnesium und Calcium sowie Amine, wie Triäthanolamin, Diäthylaminoäthanol, Triäthylamin, Trimethylamin oder Diäthylamin. Verbindungen der allgemeinen Formel I mit einem basischem Heterocyclus $R_2$ können mit starken Säuren auch pharmazeutisch annehmbare Säureadditionssalze bilden, wozu insbesondere Mineralsäuren, wie Salzsäure in Frage kommen.

Die Verbindungen der allgemeinen Formel I sowie deren Salze haben eine antiinflammatorische, analgetische und antirheumatische Wirkung. Diese wertvollen pharmakologischen Eigenschaften können unter Verwendung von Standardmethoden bestimmt werden, beispielsweise im bekannten Kaolin-Pfotenoedemtest (an der Ratte). In diesem Test wird in der rechten Hinterpfote der Ratte durch intradermale Injektion von 0,1 ml einer 10%igen Kaolinsuspension (bolus alba) eine akute lokale Entzündung erzeugt. Die zu untersuchende Substanz wird auf oralem Wege verabreicht, und die folgenden Parameter werden gemessen:

1. Durchmesser der Pfote in mm (als Ausdruck der Heftigkeit der Entzündung);
2. Druck (in g) auf die Pfote (zur Ermittlung der Schmerzschwelle).

1/2 Stunde vor und 3 1/2 Stunden nach der Kaolininjektion wird die zu untersuchende Substanz verabreicht, und 4 Stunden nach der Kaolin-Injektion werden die oben erwähnten Parameter gemessen. Der oedemhemmende Effekt wird in Prozenten angegeben, basierend auf der Differenz der Oedemintensität zwischen unbehandelten und mit der zu untersuchenden Substanz behandelten Tieren, die antinoziceptive Aktivität durch die prozentuale Erhöhung der Schmerzschwelle.

In diesem Test zeigt das 6-Chlor-4-hydroxy-2-methyl-3-(2-pyridyl-carbamoyl)-2H-thieno[2,3-e]1,2-thiazin-1,1-dioxid bei einer Dosierung von 0,3 mg/kg p.o. eine 20%ige Oedemhemmung und eine 97%ige Erhöhung der Schmerzschwelle und bei einer Dosierung von 1 mg/kg p.o. eine 33%ige Oedemhemmung und eine 129%ige Erhöhung der Schmerzschwelle.

Die Verbindungen der allgemeinen Formel I besitzen qualitativ eine ähnliche Wirkung wie Phenylbutazon, welches für seien therapeutische Verwendung und Eigenschaften bekannt ist. Ausserdem hemmen sie — wie dies in einem entsprechenden Standard-Test gezeigt werden kann — die Blutplättchenaggregation und haben demnach auch antithrombotische Eigenschaften.

Die Verbindungen der allgemeinen Formel I sowie deren Salze können als Heilmittel, z.B. in Form pharmazeutischer Präparate Verwendung finden, welche sie in Mischung mit einem für die enterale oder parenterale Applikation geeigneten, pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline usw. enthalten. Die pharmazeutischen Präparate können in fester Form, z.B. als Tabletten, Dragées, Suppositorien, Kapseln, in halbfester Form, z.B. als Salben, oder in flüssiger Forme, z.B. als Lösungsen, Suspensionen oder Emulsionen, vorliegen. Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer. Sie können auch noch andere therapeutisch wertvolle Stoffe enthalten.

Die Verbindungen der allgemeinen Formel I können beim Menschen oral in einer täglichen Dosis von 1—100 mg, vorzugsweise 2—10 mg, verabreicht werden.

Die nachfolgenden Beispiele, in welchen alle Temperaturen in Celsiusgraden angegeben sind, erläutern die Erfindung.

Beispiel 1

0,4 g (1,29 mMol) 6-Chlor-4-hydroxy-2-methyl-3-methoxycarbonyl-2H-thienol[3,2-e]1,2-thiazin-1,1-dioxid und 0,18 g (1,94 mMol) 2-Aminopyridin werden in 45 ml absolutem Xylol unter Ueberleiten eines Stickstoffstromes 16 Stunden unter Rückfluss erhitzt. Man lässt auf Raumtemperatur abkühlen und bringt das Produkt durch Anreiben zum Auskristallisieren. Es wird über Nacht in der Kälte aufbewahrt. Die ausgefallenen Kristalle werden abgesaugt und aus Dioxan umkristallisiert. Man erhält 6-Chlor-4-hydroxy-2-methyl-3-(2-pyridylcarbamoyl)-2H-thieno[3,2-e]1,2-thiazin-1,1-dioxid vom Schmelzpunkt 239—241° (Zers.).

Das in diesem Beispiel verwendete Ausgangsmaterial erhält man wie folgt:

59 g (1,48 Mol) Natriumhydroxid werden in 637 ml Wasser gelöst. Die Lösung wird auf 90°C erwärmt, worauf 290,7 g (1,48 Mol) 2,5-Dichlorthiophen-3-carbonsäure zugegeben und unter Rühren gelöst werden. Zu der Lösung werden 156 g (1,50 Mol) Natriumhydrogensulfit, gelöst in 430 ml Wasser zugegeben, und es wird mit ca. 167 ml 30%iger Natronlauge auf pH 7,5—7,7 eingestellt. Zu der klaren Lösung werden 12 g (0,12 Mol) fein zerriebenes Kupfer(I)-chlorid zugegeben, worauf 24 Stunden rückflussgekocht wird. Es wird heiss vom Kupferoxid abgesaugt und das Filtrat wird mit 400 ml konz. HCl angesäuert und auf 5° abgekühlt. Der ausgefallene Niederschlag wird ohne Nachwaschen abgesaugt und im Vakuum bei 110° bis zur Gewichtskonstanz getrocknet. Danach wird mit 3 x 500 ml Methylenchlorid ausgekocht. Der Rückstand wird bei 70° im Vakuum getrocknet. Man erhält das Natriumsalz von 5-Chlor-2-sulfothiophen-3-carbonsäure.

129 g (0,488 Mol) des Natriumsalzes von 5-Chlor-2-sulfothiophen-3-carbonsäure werden in einer Lösung von 84,7 g (1,523 Mol) Kalilauge in 430 ml Wasser heiss gelöst, mit 130 ml konz. HCl auf pH 1 angesäuert und langsam auf 5° abgekühlt. Der ausgefallene Niederschlag wird ohne Nachwaschen abgesaugt. Das Filtrat wird auf 220 ml eingeengt und abermals auf 5° abgekühlt. Der ausgefallene Niederschlag wird abgesaugt, mit dem vorher gewonnenen Produkt vereint und im Vakuum bei 110° getrocknet, Man erhält das Kaliumsalz von 5-Chlor-2-sulfothiophen-3-carbonsäure.

25 g (0,089 Mol) des Kaliumsalzes von 5-Chlor-2-sulfothiophen-3-carbonsäure werden in 100 ml Phosphoroxychlorid unter Rühren suspendiert. Zur Suspension werden 39 g (0,187 Mol) Phosphorpentachlorid gegeben, worauf 3 Stunden bei 95° gerührt wird. Anschliessend wird auf 10° abgekühlt und von ausgefallenen anorganischen Salzen abgesaugt. Das Filtrat wird im Vakuum soweit als möglich eingedampft und zur Entfernung noch vorhandener anorganischer Salze in 200 ml abs. Chloroform aufgenommen und filtriert. Das Lösungsmittel wird abdestilliert, wobei nicht kristallisierendes Oel hinterbleibt. Man erhält 5-Chlor-2-chlorsulfonylthiophen-3-carbonsäurechlorid.

21 g (0,075 Mol) 5-Chlor-2-chlorsulfonylthiophen-3-carbonsäurechlorid werden in 210 ml absolutem Chloroform gelöst, worauf 3,6 g (0,113 Mol) absolutes Methanol zugegeben werden und bis zum Ende der HCl-Entwicklung rückflussgekocht (ca. 4 Stunden) wird. Nach dem Abdestillieren des Lösungsmittels hinterbleibt ein beim Abkühlen auskristallisierendes Oel, das ohne weitere Reinigung verarbeitet werden kann. Man erhält 5-Chlor-2-chlorsulfonylthiophen-3-carbonsäuremethylester.

18,6 g (0,068 Mol) 5-Chlor-2 chlorsulfonylthiophen-3-carbonsäuremethylester und 17 g (0,135 Mol) Glycinmethylester-Hydrochlorid werden in 190 ml absolutem Pyridin 6 Stunden bei Raumtemperatur gerührt. Das Lösungsmittel wird anschliessend im Vakuum abdestilliert. Der Rückstand wird zwischen 150 ml 2 n HCl und 400 ml Aether verteilt, die organische Phase abgetrennt und die wässrige Phase mit 3 x 100 ml Aether ausgeschüttelt. Die vereinigten organischen Phasen werden mit Natriumsulfat getrocknet, mit Aktivkohle gerührt, filtriert und eingedampft. Der ölige Rückstand wird mit Aether zu Kristallisation gebracht, abgesaugt und mit wenig eiskaltem Aether digeriert. Man erhält 5-Chlor-2-(N-methoxycarbonylmethyl)-sulfamoylthiophen-3-carbonsäuremethylester von Schmelzpunkt 95—97°.

10,6 g (0,0693 g AT) Natrium werden in 100 ml absolutem Methanol gelöst. Die Lösung im Vakuum eingedampft und anschliessend im Vakuum 1 Stunde auf 100° erhitzt. 10,6 g (0,033 Mol) 5-Chlor-2-(N-methoxycarbonylmethyl)-sulfamoylthiophen-3-carbonsäuremethylester werden in 75 ml absolutem Benzol gelöst, und zu der Lösung wird das oben bereitete Natriummethylat, aufgeschlämmt in 33 ml absolutem Benzol, zugegeben und 6 Stunden bei 60° gerührt. Danach wird auf 150 ml 2 n HCl und 100 g Eis gegossen und mit 3 x 200 ml Methylenchlorid ausgeschüttelt. Die vereinigten organischen Phasen werden mit 3 x 100 ml 10%iger Natriumacetatlösung ausgeschüttelt, wobei die wässrigen Phasen jeweils mit wenig Methylenchlorid rückgeschüttelt werden. Danach werden die organischen Phasen mit 5 x 150 ml 10%iger Natriumcarbonatlösung extrahiert, wobei die wässrigen Phasen jeweils mit wenig Methylenchlorid rückgeschüttelt werden. Die vereinigten wässrigen Phasen werden mit konz. HCl auf pH 1 angesäuert, worauf das ausfallende Produkt mit 3 x 200 ml Methylenchlorid extrahiert wird. Die vereinigten oganischen Phasen werden mit Natriumsulfat getrocknet, mit Aktivkohle gerührt, filtriert und eingedampft, wobei das Produkt auskristallisiert. Man erhält 6-Chlor-4-hydroxy-3-methoxycarbonyl-2H-thieno[3,2-e]1,2-thiazin-1,1-dioxid vom Schmelzpunkt 178—179°.

1,9 g (6,43 mMol) 6-Chlor-4-hydroxy-3-methoxycarbonyl-2H-thieno[3,2-e]1,2-thiazin-1,1-dioxid werden in 19 ml absolutem Dimethylformamid gelöst und auf 0° abgekühlt. Danach werden 0,56 g (13,5 mMol) Natriumhydridsuspension in Weissöl 58%, die durch Waschen mit 2 x 10 ml absolutem benzol vom Oel befreit wurden, zugegeben und bei 0° 2 Stunden gerührt. Anschliessend werden 1,0 g (7,07 mMol) methyljodid so zugegeben, dass die Temperatur 10° nicht übersteigt, worauf 2 Stunden bei Raumtemperatur gerührt wird. Das Lösungsmittel wird im Vakuum abdestilliert, und der Rückstand wird zwischen 100 ml 2 n HCl und 200 ml Methylenchlorid verteilt. Die Phasen werden getrennt und die wässrige Phase mit 50 ml Methylenchlorid ausgeschüttelt. Die vereinigten organischen Phasen werden mit Natriumsulfat getrocknet, mit Aktivkohle gerührt, filtriert und eingedampft. Das zurückbleibende Oel wird mit Methanol zum Kristallisieren gebracht. Das Produkt wird abgesaugt und mit wenig Methanol digeriert. Man erhält 6-Chlor-4-hydroxy-2-methyl-3-methoxycarbonyl-2H-thieno[3,2-e]1,2-thiazin-1,1-dioxid vom Schmelzpunkt 173—174°.

Beispiel 2

0,4 g (1,29 mMol) 6-Chlor-4-hydroxy-2-methyl-3-methoxycarbonyl-2H-thieno[3,2-e]1,2-thiazin-1,1-dioxid werden mit 0,19 g (1,94 mMol) 2-Aminothiazol in 45 ml absolutem Xylol unter Ueberleiten eines Stickstoffstromes 16 Stunden unter Rückfluss erhitzt. Man lässt auf Raumtemperatur abkühlen, bringt das Produkt durch Anreiben zum Auskristallisieren und bewahrt über Nacht in der Kälte auf. Die ausgefallenen Kristalle werden abgesaugt und aus Aethanol umkristallisiert. Man erhält 6 - Chlor - 4 - hydroxy - 2 - methyl - 3 - (2 - thiazolcarbamoyl) - 2H - thieno[3,2-e]1,2 - thiazin - 1,1-dioxid vom Schmelzpunkt 136—138° (Zers.).

Beispiel 3

2,59 g 6-Chlor-3,4-dihydro-2-methyl-4-oxo-2H-thieno[3,2-e]1,2-thiazin-1,1-dioxid und 1,8 ml Pyrrolidin werden in 20 ml Benzol gelöst. Nach Zugabe von 3 mg p-Toluolsulfonsäure-monohydrat wird am Wasserabscheider bis zur Ansammlung von 0,2 ml Wasser unter Rückflauss erhitzt. Man engt zur Trockne ein, trocknet am Hochvakuum, nimmt den Rückstand mit wenig Benzol auf und verdünnt mit Diäthyläther und Hexan. Bei —25° kristallisieren kubische, rotbraune Kristalle von 6-Chlor-2-methyl-4-(1-pyrrolidino)-2H-thieno[3,2-e]1,2-thiazin-1,1-dioxid vom Smp. 150—151,5° aus.

Eine Lösung von 1,34 g 6-Chlor-2-methyl-4-(1-pyrrolidino)-2H-thieno[3,2-e]1,2-thiazin-1,1-dioxid und 0,8 ml Triäthylamin in 25 ml einer 4:1-Mischung von Tetrahydrofuran und Benzol wird während 1 Stunde zu einer gerührten und auf —10° gekühlten Mischung von 0,5 g Phosgen, 6 ml Benzol und 3 ml Tetrahydrofuran getropft. Man lässt 15 Stunden bei —25° stehen, erwärmt auf 20° und gibt unter Rühren eine Lösung von 0,64 g 2-Aminopyridin und 0,8 ml Triäthylamin in 5 ml Tetrahydrofuran tropfenweise während 30 Minuten zu. Man erhitzt weitere 2 Stunden unter Rückfluss, trägt auf Eiswasser auf, extrahiert mit Methylenchlorid, dampft die organische Phase zur Trockne eine und chromatographiert den Rückstand an Kieselgel. Mit Methylenchlorid-Aethylacetat 9:1 eluiert, kristallisiert nach dem Eindampfen das 6-Chlor-2-methyl-3-(2-pyridyl-carbamoyl)-4-(1-pyrrolidino)-2H-thieno[3,2-e]1,2-thiazin-1,1-dioxid aus Diäthyläther als gelbe hexagonale Kristalle vom Smp. 167,5—168° aus.

0,5 g 6-Chlor-2-methyl-3-(2-pyridyl-carbamoyl)-4-(1-pyrrolidino)-2H-thieno[3,2-e]1,2-thiazin-1,1-dioxid werden in 15 ml 2 n Salzsäure während 15 Minuten unter Rückfluss erhitzt. Man entfernt den Hauptteil der Salzsäure am Vakuum, trägt den Rückstand auf Phosphatpufferlösung von pH 6 aus und extrahiert mehrmals mit Methylenchlorid. Das Lösungsmittel wird am Vakuum entfernt, und der feste gelbe Rückstand aus Dioxan umkristallisiert. Man erhält 6-Chlor-4-hydroxy-2-methyl-3-(2-pyridyl-carbamoyl)-2H-thieno[3,2-e]1,2-thiazin-1,1-dioxid als gelbes Kristallpulver vom Smp. 238—239°.

Beispeil 4

In einem 4 1-Kolben wird ein Gemisch aus 1230 g (10,6 Mol) Chlorsulfonsäure und 1000 g (8,45 Mol) Thionylchlorid vorgelegt und mittels Eisbad auf 5° abgekühlt. Dann werden vorsichtig 647 g (4,23 Mol) 2,5-Dichlorthiophen so langsam zugetropft, dass die Temperatur des Reaktionsgemisches 7° nicht überschreitet. Dabei tritt heftige Gasentwicklung ein. Nach Beendigung der Zugabe wird die Reaktionslösung 1,5 Stunden bei 15° weiter gerührt. Anschliessend wird die erhaltene leicht rötliche Lösung vorsichtig auf Eis gegossen. Die so erhaltene Mischung wird noch einige Minuten durchgerührt, gröbere Brocken des kristallin anfallenden Sulfochlorids werden zerrieben. Nach Entfernen des überschüssigen Eieses wird der Niederschlag abgesaugt, mit dest. Wasser gewaschen und ausgepresst. Das so erhaltene rohe, rötliche Sulfochlorid wird in 4000 ml Methylenchlorid aufgenommen und die Lösung mit wasserfreiem Magnesiumsulfat getrocknet. Fällt nach dem Zersetzen mit Eis das Sulfochlorid als Oel an, so extrahiert man die wässrige Phase mit Methylenchlorid und verfährt wie beschrieben. Nach dem Eindampfen der klaren Methylenchloridphase wird ein rötliches Oel erhalten, das an der Wasserstrahlpumpe von restlichem Lösungsmittel befreit und anschliessend in den Eiskasten (—20°) gestellt wird. Man erhält 2,5-Dichlorthiophen-3-sulfonsäurechlorid in Form derber Kristalle, welches ohne weitere Reinigung in die nächste Stufe eingesetzt werden kann. Nach Umkristallisation aus Petroläther zeigt das Produkt den Smp. 25—27°.

3500 ml absolutes Chloroform werden in einem 4 1-Kolben vorgelegt und 533 g (2,12 Mol) 2,5-Dichlorthiophen-3-sulfonsäurechlorid werden darin gelöst. Unter Rühren wird bei 20° solange trockenes Methylamin eingeleitet, bis die Lösung mit angefeuchtetem Indikatorpapier basisch reagiert. Die Lösung wird noch 1—1,5 Stunden bei Raumtemperatur gerührt, dann im Scheidetrichter mit 0,5 n HCl mehrmals extrahiert, mit wasserfreiem Natriumsulfat getrocknet und im Vakuum eingedampft. Das so erhaltene Oel wird ins Tiefkühlfach gestellt, wo es kristallisiert. Zur Reinigung wird das so erhaltene Produkt aus 1500 ml Tetrachlorkohlenstoff umkristallisiert und im Vakuumtrockenschrank bei 40° getrocknet. Man erhält 2,5-Dichlor-thiophen-3-sulfonsäuremethylamid vom Smp. 59—61°.

250 g (1,016 Mol) 2,5-Dichlor-thiophen-3-sulfonsäuremethylamid werden in einem 4 1-Kolben in 1500 ml absolutem Aether gelöst, die Apparatur mit trockenem Stickstoff gespült und langsam eine Lösung von 2,54 Mol n-Butyllithium in 2200 ml Aether unter Rühren und Beibehaltung der Stickstoffspülung innerhalb 1 Stunde zugetropft. Die Lösung erwärmt sich dabei zum Sieden und ein weisser Niederschlag fällt aus, der allmählich einen grauen Farbton anzunehmen beginnt. Die Lösung wird nach beendeter Butyllithium-Zugabe 5 Stunden rückflussgekocht, nach denm Abkühlen auf etwa 25° wird unter Rühren trockenes Kohlendioxyd 1/2 Stunde in die Suspension eingeleitet. Danach wird die Suspension auf 2000 ml Wasser gegossen und solange konz. Salzsäure zugesetzt, bis die wässrige Phase stark sauer reagiert. Nun wird die ätherische Phase, in der sich Ausgangs- und Endprodukt befinden, im Scheidetrichter abgetrennt und die wässrige Phase zusätzlich mit Aether extrahiert. Die vereinigten Aetherphasen werden 3 x mit je 300 ml 5% Natriumhydrogencarbonat-Lösung extrahiert. Die vereinigten Natriumhydrogencarbonat-Phasen werden mit konz. Salzsäure angesäuert (pH 1—2) und mit 4000 ml Aether extrahiert. Die Aetherphase wird mit wasserfreiem Magnesiumsulfat getrocknet und eingedampft. Die rohe, leicht gelblich gefärbte Carbonsäure kristallisiert dabei sofort. Zur Reinigung wird das erhaltene Produkt aus Eisessig umkristallisiert. Man erhält 5-Chlor-3-methylsulfamoylthiophen-2-carbonsäure.

In einem 4 1-Kolben werden 1500 ml absolutes Chloroform vorgelegt und unter Rühren darin 90 g (0,352 Mol) feinpulverisierte 5-Chlor-3-methylsulfamoylthiophen-2-carbonsäure suspendiert. Dann werden 85,17 g (0,41 Mol) Phosphorpentachlorid zugegeben und die Lösung auf 35° erwärmt. Nach etwa 15 Minuten entsteht eine klare Lösung. Höhere Temperaturen müssen unbedingt vermieden werden, da sonst das Ausgangsprodukt ringschliesst. Nach insgesamt 20—30 Minuten Rühren wird die Heizung ausgeschaltet, der Tropftrichter aufgesetzt und 250 ml absolutes Methanol in die Lösung eingetropft. Nach beendeter Methanolzugabe wird die Lösung 10 Minuten rückflussgekocht und dann erkalten gelassen. In Scheidetrichter wird die Chloroform-Phase mehrmals mit wenig Natriumhydrogencarbonat-Lösung (5%) extrahiert, mit Wasser gewaschen, mit wasserfreiem Natriumsulfat getrocknet und dann im Vakuum eingedampft. Beim Anreiben des erhaltenen Oeles kristallisiert dieses. Zur Reinigung wird das so erhaltene Rohprodukt aus Methanol umkristallisiert. Man erhält 5-Chlor-3-methylsulfamoylthiophen-2-carbonsäuremethylester vom Smp. 103—104°.

9,7 g (0,2224 Mol) einer 55% Natriumhydridsuspension in Weissöl werden in einer Glassinternutsche mit abs. Benzol gewaschen und in einem 1 Liter-Kolben unter Stickstoffatmosphäre in 70 ml abs. Dimethylformamid suspendiert. Dann werden unter Rühren 60 g (0,2224 Mol) 5-Chlor-3-methylsulfamoylthiophen-2-carbonsäuremethylester in 525 ml absolutem Dimethylformamid gelöst bei 0° innerhalb 1 Stunde zugetropft. Anschliessend wird 20 Minuten gerührt und dann eine Lösung von 44,5 g (0,2224 Mol) Jodessigsäuremethylester in 65 ml abs. Dimethylformamid bei 0—5° unter Kühlung innerhalb von 2 Stunden zugetropft. Die Lösung wird nun noch so lange bei Raumtemperatur gerührt, bis ein angefeuchtetes Indikatorpapier neutrale Reaktion zeigt. Anschliessend wird im Vakuum (ca. 1 mm) das Dimethylformamid abdestilliert und der Rückstand zwischen 400 ml 0,5 n Salzsäure und 400 ml Methylenchlorid verteilt. Die Methylenchloridphase wird im Scheidetrichter abgetrennt, die Wasserphase mit wenig Methylenchlorid extrahiert und die vereinigten organischen Phasen zweimal mit 150 ml 5% Natriumhydrogencarbonat-Lösung augeschüttelt. Anschliessend wird die Methylenchloridphase mit wasserfreiem Natriumsulfat getrocknet und im Vakuum eingedampft. Dabei wird ein kristalliner Rückstand erhalten, der durch Umkristallisation aus Methanol gereinigt wird. Man erhält 5-Chlor-3-(N-methoxycarbonyl-N-methyl)-sulfamoylthiophen-2-carbonsäuremethylester vom Smp. 103—104°.

170 ml absolutes Methanol werden in einem 0,5 1-Kolben vorgelegt und dieser mit getrocknetem Stickstoff gespült. Dann werden unter Rühren portionsweise 3,53 g Natrium (0,146 Mol) eingetragen. Es wird die totale Auflösung des Natriums abgewartet, worauf man auf ca. 30° abkühlen lässt und anschliessend unter Rühren 50 g 5-Chlor-3-(N-methoxycarbonyl-N-methyl)-sulfamoylthiophen-2-carbonsäuremethylester (0,146 Mol) einträgt. Die Suspension wird 15 Minuten bei 30° gerührt und dann vorsichtig zum Sieden erwärmt. Dabei entsteht eine klare, orange gefärbte Lösung. Das Sieden wird solange beibehalten bis gemäss Dünnschichtchromatogram alles Ausgangsmaterial umgesetzt ist (Dauer etwa 25—35 Minuten). Nach dem Abkühlen auf ca. 45° wird auf 200 g Eis/300 ml 2 n Salzsäure gegossen, wobei das Produkt ausfällt. Im Scheidetrichter wird die entstandene wässrige Suspension mit 2 x 400 ml Methylenchlorid mehrmals ausgeschüttelt. Die vereinigten Methylenchloridphasen werden mit 2 x 150 ml 10% Natriumacetatlösung extrahiert und anschliessend mit 3 x 100 ml 10% Natriumcarbonatlösung ausgeschüttelt. Die vereinigten Natriumcarbonatphasen werden mit wenig Methylenchlorid gewaschen und mit konz. Salzsäure unter gutem Rühren auf pH 1—2 angesäuert. Die wässrige Phase, die das ausgefallene Endprodukt enthält, wird mit 2 x 500 ml Methylenchlorid extrahiert. Die vereinigten Methylenchloridphasen werden mit Natriumsulfat getrocknet und im Vakuum eingedampft, Man erhält 6-Chlor-4-hydroxy-2-methyl-3-methoxycarbonyl-2H-thieno[2,3-e]1,2-thiazin-1,1-dioxid als gelbe, körnige Kristalle, die nach Digerieren in wenig kaltem Methanol, Absaugen und Trocknen im Vakuumtrockenschrank bei 70° rein genug für die weitere Umsetzung sind. Für die Elementaranalyse wird eine kleine Menge aus Benzol umkristallisiert, die einen Smp. von 200—203° (Zers.) zeigt.

In einem 2 1-Kolben werden 1200 ml absolutes Xylol vorgelegt und unter Rühren 11 g 6-Chlor-4-hydroxy-2-methyl-3-methoxycarbonyl-2H-thieno[2,3-e]1,2-thiazin-1,1-dioxid (0,0355 Mol) zugegeben. Anschliessend werden 4,42 g 2-Aminopyridin (0,0469 Mol) eingetragen und die Lösung anschliessend rückflussgekocht, bis gemäss Dünnschichtchromatogram alles Ausgangsprodukt umgesetzt ist (Dauer 4 Stunden). Während des Rückflusskochens wird ein schwacher Stickstoffstrom durchgeleitet, um so das entstehende Methanol auszutragen. Nach vollendeter Umsetzung lässt man auf ca. 120° abkühlen, setzt Aktivkohle zu und kocht noch einmal kurz auf. Ein Heisswassertrichter wird mit Glycerin gefüllt und auf 120° vorgeheizt, die 120° heisse Reaktionslösung dann über diesen Trichter filtriert. Wenn die Temperatur der Lösung auf 70° gesunken ist, wird angerieben, worauf sich kleine orange Kristalle abzuscheiden beginnen. Nach dem Erkalten wird die Lösung noch 12 Stunden im Eiskasten (—5°) aufbewahrt und der ausgefallene Niederschlag abgesaugt und mit Xylol gewaschen. Die Trocknung erfolgt im Vakuum (1 mm, 120°, 2 Stunden). Die so erhaltenen orange bis gelben Kristalle Stellen 6-Chlor-4-hydroxy-2-methyl-3-(2-pyridyl-carbamoyl)-2H-thieno[2,3-e]1,2-thiazin-1,1-dioxid vom Smp. 225—230° (Zers.) dar.

## Beispiel 5

268,5 g fein gepulvertes Natriumsalz der 2,5-Dibromthiophen-3-sulfonsäure (0,78 Mol) werden in 400 ml Phosphoroxychlorid aufgeschlämmt, 171 g Phosphorpentachlorid (0,82 Mol) zugegeben und 2 1/2 Stunden auf 90° erhitzt. Dann wird im Vakuum scharf eingedampft, der Rückstand auf 600 g Eis gegossen, mit 3 x 250 ml Chloroform extrahiert, die organische Phase mit 2 x 100 ml Wasser gewaschen, getrocknet und eingedampft. Das zurückbleibende dunkle Oel bestehend aus 2,5-Dibromthiophen-3-sulfonsäurechlorid ist für die weitere Umsetzung rein genug.

150 g (0,44 Mol) 2,5-Dibromthiophen-3-sulfonsäurechlorid werden in 500 ml absolutem Chloroform gelöst. In die Lösung wird bei 20—25° so lange trockenes Methylamin eingeleitet, bis ein angefeuchtetes pH-Papier mit der Lösung basisch reagiert. Nach einer weiteren Stunde Rühren wird das Reaktionsgemisch mehrmals mit 0,5 n Salzsäure extrahiert, mit Wasser gewaschen und die organische Phase über Natriumsulfat getrocknet. Nach Eindampfen der organischen Phase im Vakuum verbleibt ein kristalliner Rückstand, der mit wenig Diisopropyläther digeriert wird. Man erhält N-Methyl-2,5-dibrom-thiophen-3-sulfonamid vom Smp. 100—104°.

Zu einer Lösung von 33,5 g N-Methyl-2,5-dibrom-thiophen-3-sulfonamid (0,1 Mol) in 520 ml absolutem Aether wird bei Raumtemperatur eine Lösung von N-Butyllithium, hergestellt aus 223 ml Aether, 24,3 g N-Butylbromid und 4,4 g Lithium, getropft. Die Lösung wird noch 2 Stunden bei Raumtemperatur gerührt und dann trockenes Kohlendioxyd eingeleitet. Das Reaktionsgemisch wird auf Wasser gegossen, die Aetherphase abgetrennt, die wässrige Phase mit Salzsäure angesäuert und das Produkt mit Aether extrahiert. Nach Eindampfen der getrockneten Aetherphase bleibt ein kristallines

Produkt zurück, das mit Benzol digeriert wird. Man erhält 5-Brom-3-N-methylsulfamoylthiophen-2-carbonsäure vom Smp. 165—185°.

30,0 g (0,1 Mol) 5-Brom-3-N-methylsulfamoylthiophen-2-carbonsäure werden in 400 ml absolutem Chloroform aufgeschlämmt und unter Kühlen werden bei 5—10° 24,2 g Phosphorpentachlorid (0,11 Mol) zugesetzt. Sobald eine klare Lösung entstanden ist, wird noch 20 Minuten bei 10° weiter gerührt und dann 100 ml abs. Methanol zugetropft. Es wird 10 Minuten rückflussgekocht, dann die Lösung mit Natriumhydrogencarbonat-Lösung extrahiert, getrocknet und eingedampft. Das zurückbleibende Oel wird mit Methanol angerieben. Man erhält 5-Brom-3-N-methylsulfamoyl-thiophen-2-carbonsäuremethylester vom Smp. 114—117°.

Zu einer Suspension von 2,52 g Natriumhydrid (0,105 Mol) in 12 ml abs. Dimethylformamid wird bei 0° die Lösung von 31,4 g (0,1 Mol) 5-Brom-3-N-methylsulfamoyl-thiophen-2-carbonsäuremethylester in 160 ml absolutem Dimethylformamid getropft. Es wird noch 1 Stunde bei 0—2° gerührt und dann bei 0—5° eine Lösung von 16,1 g (0,105 Mol) Bromessigsäuremethylester in 20 ml absolutem Dimethylformamid zugetropft und noch 3 Stunden bei Raumtemperatur gerührt. Die Reaktionslösung wird im Vakuum eingedampft, der Rückstand zwischen Methylenchlorid und 2 n Salzsäure verteilt, die organische Phase abgetrennt, mit Bicarbonatlösung und Wasser gewaschen, getrocknet und eingedampft. Der kristalline Rückstand wird mit wenig Tetrachlorkohlenstoff digeriert. Man erhält 5-Brom-3-(N-methoxycarbonylmethyl-N-methyl)-sulfamoylthiophen-2-carbonsäuremethylester von Smp. 98—101°.

In 15 ml einer methanolischen Lösung von einem Hundertstel Mol Natriummethylat werden unter Rühren portionsweise 3,86 g (0,01 Mol) 5-Brom-3-(N-methoxycarbonylmethyl-N-methyl)-sulfamoyl-thiophen-2-carbonsäuremethylester zugegeben, 15 Minuten bei Raumtemperatur gerührt und dann 20 Minuten rückflusserhitzt. Die Lösung wird auf Eis und 2 n Salzsäure gegossen und das Produkt mit Methylenchlorid extrahiert. Die organische Phase wird mit 10%iger Natriumacetatlösung gewaschen und dann mit 10%iger Natriumcarbonatlösung extrahiert. Die Natriumcarbonatlösung wird mit Salzsäure angesäuert und das ausfallende Produkt mit Methylenchlorid ausgeschüttelt. Nach Eindampfen der getrockneten organischen Phase erhält man ein kristallines Produkt, das mit wenig Methanol digeriert wird. Man erhält 6-Brom-4-hydroxy-2-methyl-3-methoxycarbonyl-2H-thieno[2,3-e]1,2-thiazin-1,1dioxid vom Smp. 196—201°.

3,54 g (0,01 Mol) 6-Brom-4-hydroxy-2-methyl-3-methoxycarbonyl-2H-thieno[2,3-e]1,2-thiazin-1,1-dioxid werden zusammen mit 1,42 g (0,015 Mol) 2-Aminopyridin in 150 ml absolutem Xylol 6 Stunden rückflussgekocht. Man lässt auf Raumtemperatur abkühlen und kristallisiert das ausfallende Rohprodukt aus Dioxan um. Man erhält 6-Brom-4-hydroxy-2-methyl-3-(2-pyridyl-carbamoyl)-2H-thieno[2,3-e]1,2-thiazin-1,1-dioxid vom Smp. 232—235°.

Beispiel 6

40 g (0,2 Mol) 4-Acetylamino-3-thiophencarbonsäuremethylester werden in 1 Liter absoluten Chloroform gelöst, worauf bei 20—25° eine Lösung von 27 g (0,2 Mol) Sulfurylchlorid in 100 ml abs. Chloroform innerhalb 20 Minuten zugetropft wird. Hierauf wird 1 Stunde unter Rückfluss gekocht. Die erhaltene bräunliche Lösung wird abgekühlt und bei 10° mit 1 Liter Eiswasser versetzt. Die organische Lösung wird dann mit 200 ml 5%iger Natriumbicarbonatlösung gewaschen, über Natriumsulfat getrocknet und im Vakuum zur Trockne eingeengt. Der ölige Rückstand wird mit 100 ml Aether angerieben, wobei Kristallisation eintritt. Das Produkt wird abgenutscht und aus Essigester umkristallisiert; man erhält 4-Acetylamino-5-chlor-3-thiophencarbonsäuremethylester vom Smp. 119—121°. Das ätherische Filtrat und die Mutterlauge der Umkristallisation aus Essigester werden vereinigt, zur Trockene eingedampft und das zurückbleibende Oel an 300 g Kieselgel mit Methylenchlorid/Essigester 4:1 als Eluiermittel chromatographiert. Man erhält eine zusätzliche Portion von 4-Acetylamino-5-chlor-3-thiophencarbonsäuremethylester vom Smp. 119—121°.

35 g 4-Acetylamino-5-chlor-3-thiophencarbonsäuremethylester werden mit 350 ml einer 5 n Lösung von Chlorwasserstoff in abs. Methanol 30 Minuten unter Rückfluss gekocht. Die dunkel gefärbte Lösung wird dan nach üblicher Weise mit Aktivkohle behandelt, abfiltriert und im Vakuum zur Trockene eingedampft. Der kristalline Rückstand wird nun in 180 ml absolutem Methanol bei Raumtemperatur gelöst und die erhaltene Lösung unter ständigem Rühren portionenweise mit 1400 ml absolutem Aether versetzt. Nach 30-minütigem Abkühlen im Eisbad wird das auskristallisierte, fast farblose Produkt abgenutscht, mit Aether gewaschen und 1 Stunde an der Luft getrocknet. Man erhält 4-Amino-5-chlor-3-thiophencarbonsäuremethylester-hydrochlorid, welches sich zwischen 120° und 140° zersetzt. Dieses Hydrochlorid kann ohne weitere Reinigung für die nächste Stufe eingesetzt werden.

22,8 g (0,1 Mol) 4-Amino-5-chlor-3-thiophencarbonsäuremethylester-hydrochlorid werden in 100 ml 36%iger Salzsäure suspendiert. Bei —10° wird eine Lösung von 7,25 g (0,105 Mol) Natriumnitrit in 20 ml Wasser unter die Oberfläche des Reaktionsgemisches innerhalb 10 Minuten zugetropft. Man lässt 30 Minuten —10° nachreagieren und versetzt mit einem Gemisch, das 150 ml einer 30%igen Lösung von Schwefeldioxid in Eisessig und einer Lösung von 7,5 g Kupfer(II)-chlorid in 10 ml Wasser unmittelbar vorher zubereitet wurde. Das Gemisch wird 1 Stunde bei 0—5° und 3 Stunden bei Raumtemperatur weitergerührt und dann in 21 Eiswasser gegossen. Das auskristallisierte Produkt wird

abgenutscht und in 800 ml Toluol gelöst. Die toluolische Lösung wird mit Wasser, mit einer Natriumbicarbonatlösung und schliesslich wiederum mit Wasser gewaschen. Nach dem Trocknen über Natriumsulfat und dem Einengen unter vermindertem Druck wird das zurückbleibende Oel mit wenig Hexan angerieben und über Nacht im Eiskasten stehen gelassen. Man erhält 3-Carbomethoxy-5-chlor-thiophen-4-sulfochlorid in Form von fast farblosen Kristallen, Smp. 65—67°. Umkristallisation aus Hexan erhöht den Smp. auf 67—68°.

Zu einer Lösung von 16,5 g (0,06 Mol) 3-Carbomethoxy-5-chlor-thiophen-4-sulfochlorid in 300 ml absolutem Pyridin werden bei 10—15° 7,5 g (0,06 Mol) Glycinmethylester-hydrochlorid portionenweise innerhalb 5 Minuten zugegeben. Das Reaktionsgemisch wird hierauf 4 Stunden bei 25° gerührt und dann im Vakuum zur Trockne eingeengt. Der Rückstand wird in methylenchlorid gelöst und die Lösung mit eiskalter 2 n Salzsäure ausgeschüttelt. Die organische Phase wird über Natriumsulfat getrocknet, im Vakuum eingedampft und das zurückbleibende Oel an 350 g Kieselgel mit Methylenchlorid/Essigester 4:1 als Eluiermittel chromatographiert. Die homogenen Fraktionen werden vereinigt, eingeengt und der Rückstand aus Methylenchlorid/Hexan umkristallisiert. Man erhält N-(3-Carbomethoxy-5-chlor-thiophen-4-sulfonyl)-glycinmethylester vom Smp. 68—70°.

Eine Lösung von 1,15 g (0,05 Mol) Natrium in 24 ml Methanol wird im Vakuum anschliessend im Hochvakuum zur Trockne eingedampft und der Rückstand mit 240 ml abs. Toluol übergossen. Danach werden 8,2 g (0,025 Mol) N-(3-Carbomethoxy-5-chlor-thiophen-4-sulfonyl)-glycinmethylester zugegeben und das Reaktionsgemisch während 4 Stunden bei 60° gerührt. Es wird dann auf 0° abgekühlt und unter Rühren auf 240 ml eiskalte 2 n Salzsäure gegossen. Der dabei ausgefallene Niederschlag wird abgenutscht, mit Wasser neutral gewaschen und bei 60° im Vakuum getrocknet. Das erhaltene Produkt wird mit 30 ml Methanol digeriert, erneut ab genutscht und getrocknet. Man erhält 3-Carbomethoxy-7-chlor-4-hydroxy-2H-thieno[3,4-e]1,2-thiazin-1,1-dioxid, welches zwischen 230 und 235° unter Zersetzung schmilzt.

Eine Lösung von 2,93 g (0,01 Mol) 3-Carbomethoxy-7-chlor-4-hydroxy-2H-thieno[3,4-e]1,2-thiazin-1,1-dioxid in 75 ml Dimethylformamid wird unter Argon bei 10° mit 0,022 Mol Natriumhydrid (1,0 g einer 55%igen Dispersion in Mineralöl) versetzt und hierauf 4 Stunden bei 25° gerührt. Danach werden bei 10—15° 2,5 ml (0,04 Mol) Methyljodid zugetropft. Man rührt anschliessend 16 Stunden bei Raumtemperatur, giesst dann das Reaktionsgemisch auf 750 ml Eiswasser und stellt mit konzentrierter Salzsäure sauer. Der dabei ausgefallene Niederschlag wird abgenutscht, in Chloroform gelöst und die Lösung über Natriumsulfat getrocknet. Nach dem Einengen in Vakuum wird der Rückstand an 150 g Kieselgel mit Chloroform als Eluiermittel chromatographiert. Nach Kristallisation aus Acetonitril erhält man 3-Carbomethoxy-7-chlor-4-hydroxy-2-methyl-2H-thieno[3,4-e]1,2-thiazin-1,1-dioxid vom Smp. 188—191°.

Ein Gemisch aus 2,2 g (0,07 Mol) 3-Carbomethoxy-7-chlor-4-hydroxy-2-methyl-2H-thieno[3,4-e]1,2-thiazin-1,1-dioxid, 0,9 g (0,09 Mol) 2-Aminopyridin und 240 ml abs. o-Xylol wird unter Rühren 7 Stunden unter Rückfluss erhitzt, wobei ca. 80 ml o-Xylol während der 2 ersten Stunden langsam abdestilliert werden. Nach dem Abkühlen auf Raumtemperatur wird das Reaktionsgemisch im Vakuum auf ca. 60 ml eingeengt und im Eisbad abgekühlt. Das auskristallisierte Produkt wird abgenutscht, mit Aether gewaschen, getrocknet und aus Acetonitril umkristallisiert. Man erhält 7-Chlor-4-hydroxy-2-methyl-3-(2-pyridyl-carbamoyl)-2H-thieno[3,4-e]1,2-thiazin-1,1-dioxid vom Smp. 218—220° (Zers.).

Beispiel A

Es werden in üblicher Weise Suppositorien folgender Zusammensetzung hergestellt:

| | |
|---|---|
| 6-Chlor-4-hydroxy-2-methyl-3-(2-pyridyl-carbamoyl)-2H-thieno-[2,3-e]1,2-thiazin-1,1-dioxid | 0,005 g |
| Hydriertes Kokosnussöl | 1,250 g |
| Carnaubawachs | 0,045 g |

Beispiel B

Es werden in üblicher Weise Tabletten folgender Zusammensetzung hergestellt:

| | Pro Tablette |
|---|---|
| 6-Chlor-4-hydroxy-2-methyl-3-(2-pyridyl-carbamoyl)-2H-thieno-[2,3-e]1,2-thiazin-1,1-dioxid | 5,00 mg |
| Lactose | 84,50 mg |
| Maisstärke | 10,00 mg |
| Magnesiumstearat | 0,50 mg |

Beispiel C

Es werden in üblicher Weise Kapseln folgender Zusammensetzung hergestellt:

| | | Pro Kapsel |
|---|---|---|
| 6-Chlor-4-hydroxy-2-methyl-3-(2-pyridyl-carbamoyl)-2H-thieno-[2,3-e]1,2-thiazin-1,1-dioxid | | 10 mg |
| Lactose | | 165 mg |
| Maisstärke | | 30 mg |
| Talk | | 5 mg |
| | Gesamtgewicht | 210 mg |

**Patentansprüche**

1. Thienothiazinderivate der allgemeinen Formel I

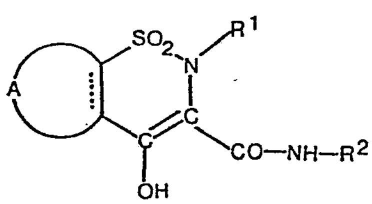

(I)

worin A mit den beiden Kohlenstoffatomen des Thiazinringes die Gruppe

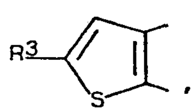
,
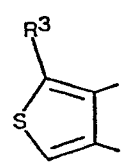
oder
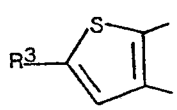

bildet und die gestrichelte Linie die im ersten und letzten Fall vorliegende Doppelbindung anzeigt, $R^1$ niederes Alkyl bedeutet, $R^2$ 2-Thiazolyl, 4-Methyl-2-thiazolyl, 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl oder einen gegebenenfalls durch Halogen, Hydroxy, niederes Alkyl, Trifluormethyl oder niederes Alkoxy substituierten Phenylrest bedeutet und $R^3$ Halogen bedeutet,

sowie Salze davon.

2. Verbindung der in Anspruch 1 difinierten allgemeinen Formel I, worin A die Gruppe

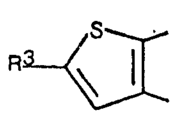

bedeutet und $R^3$ Halogen bedeutet.

3. Verbindungen gemäss Anspruch 1, worin $R^1$ Methyl, $R^2$ 2-Pyridyl und $R^3$ Chlor oder Brom bedeuten.

4. 6 - Chlor - 4 - hydroxy - 2 - methyl - 3 - (2 - pyridyl - carbamoyl) - 2H - thieno[3,2 - e]1,2 - thiazin - 1,1 - dioxid.

5. 6 - Chlor - 4 - hydroxy - 2 - methyl - 3 - (2 - pyridyl - carbamoyl) - 2H - thieno[2,3 - e]1,2 - thiazin - 1,1 - dioxid.

6. Verfahren zur Herstellung von Thienothiazinderivaten der in Anspruch 1 definierten allgemeinen Formel I und ihren Salzen, dadurch gekennzeichnet, dass man

a) eine Verbindung der allgemeinen Formel II

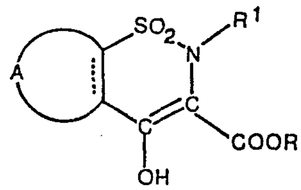

(II)

worin R niederes Alkyl bedeutet und A und $R^1$ die in Anspruch 1 genannte Bedeutung haben, mit einem Amin der allgemeinen Formel III

$$H_2N—R^2 \qquad (III)$$

worin $R^2$ die in Anspruch 1 genannte Bedeutung hat, umsetzt, oder

b) ein reaktionsfähiges funktionelles Derivat einer Säure der allgemeinen Formel IV

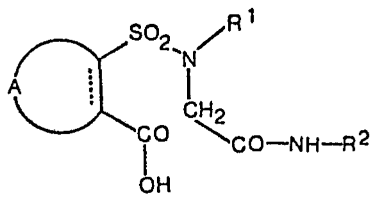

IV

worin A, $R^1$ und $R^2$ die in Anspruch 1 genannte Bedeutung haben, cyclisiert, oder dass man

c) eine Verbindung der allgemeinen Formel V

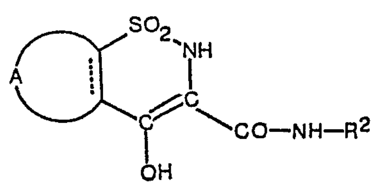

(V)

worin A und $R^2$ die in Anspruch 1 genannte Bedeutung haben, mit einem den Rest $R^1$ liefernden Mittel alkyliert, oder

d) eine Verbindung der allgemeinen Formel VI

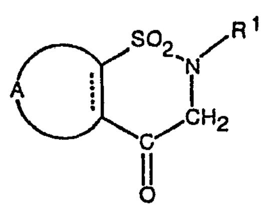

(VI)

worin A und $R^1$ die in Anspruch 1 genannte Bedeutung haben, in Gegenwart einer starken Base mit einem Isocyanat der allgemeinen Formel VII

$$O{=}C{=}N—R^2 \qquad (VII)$$

worin $R^2$ die in Anspruch 1 genannte Bedeutung hat, umsetzt, oder

e) ein Enamin der allgemeinen Formel VIII

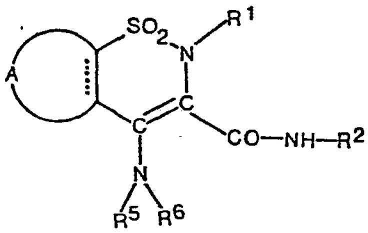

VIII

worin A, $R^1$ und $R^2$ die in Anspruch 1 genannte Bedeutung haben, und $R^5$ und $R^6$ jed nieder Alkyl bedeuten oder gemeinsam mit dem Stickstoffatom einen Pyrrolino-, Pyrrolidino-, Piperidino-, Morpholino-, oder N-(nieder Alkyl)-piperazino rest bilden,

hydrolysiert, und

f) erwünschtenfalls eine so erhaltene Verbindung der allgemeinen Formel I in ein pharmazeutisch annehmbares Salz überführt.

7. Pharmazeutisches Präparat mit anti-inflammatorischen, analgetischen, anti-rheumatischen und anti-thrombotischen Eigenschaften, enthaltend ein Thienothiazinderivat der in Anspruch 1 definierten allgemeinen Formel I oder ein Salz davon und einen Träger.

## Claims

1. Thienothiazine derivatives of general formula I

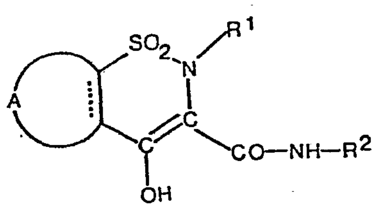

(I)

wherein A forms with the two carbon atoms of the thiazine ring the group

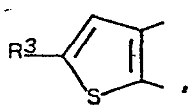

,

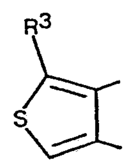

or

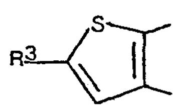

and the dotted line indicates the double bond present in the first and last case, $R^1$ signifies lower alkyl, $R^2$ signifies 2-thiazolyl, 4-methyl-2-thiazolyl, 4,5-dimethyl-2-thiazolyl, 5-methyl-1,3,4-thiadiazolyl, 2-pyrazinyl, 2-pyrimidinyl, 1,2,4-triazin-3-yl, 2-pyridyl, 3-pyridyl, 4-pyridyl, 3-methyl-2-pyridyl, 4-methyl-2-pyridyl, 5-methyl-2-pyridyl, 6-methyl-2-pyridyl, 4,6-dimethyl-2-pyridyl, 5-isoxazolyl, 5-methyl-3-isoxazolyl, 3,4-dimethyl-5-isoxazolyl, 2,6-dimethyl-4-pyrimidinyl, 1,2,3,4-tetrazol-5-yl or a phenyl group optionally substituted by halogen, hydroxy, lower alkyl, trifluoromethyl or lower alkoxy and $R^3$ signifies halogen,

as well as salts thereof.

2. Compound of general formula I defined in claim 1, wherein A signifies the group

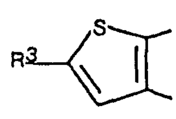

and $R^3$ signifies halogen.

3. Compounds in accordance with claim 1, wherein $R^1$ signifies methyl, $R^2$ signifies 2-pyridyl and $R^3$ signifies chlorine or bromine.

4. 6-Chloro-4-hydroxy-2-methyl-3-(2-pyridyl-carbamoyl)-2H-thieno[3,2-e]1,2-thiazine 1,1-dioxide.

5. 6-Chloro-4-hydroxy-2-methyl-3-(2-pyridyl-carbamoyl)-2H-thieno[2,3-e]1,2-thiazine 1,1-dioxide.

6. Process for the manufacture of thienothiazine derivatives of general formula I defined in claim 1 and their salts, characterised by

a) reacting a compound of general formula II

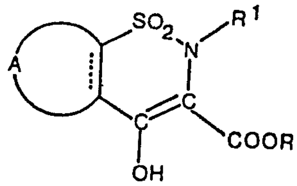

(II)

wherein R signifies lower alkyl and A and $R^1$ have the significance stated in claim 1, with an amine of general formula III

$$H_2N—R^2 \qquad \text{(III)}$$

wherein $R^2$ has the significance stated in claim 1,

or

b) cyclising a reactive functional derivative of an acid of general formula IV

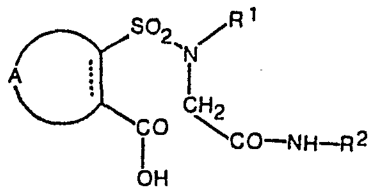

(IV)

wherein A, $R^1$ and $R^2$ have the significance stated in claim 1,

or

c) alkylating a compound of general formula V

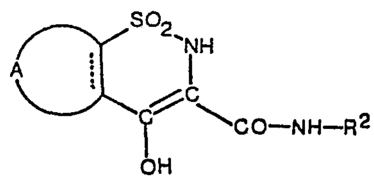

(V)

wherein A and $R^2$ have the significance stated in claim 1, with an agent yielding the group $R^1$, or

d) reacting a compound of general formula VI

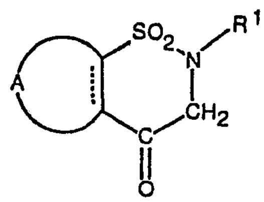

(VI)

wherein A and $R^1$ have the significance stated in claim 1, in the presence of a strong base with an isocyanate of general formula VII

$$O=C=N—R^2 \qquad \text{(VII)}$$

wherein $R^2$ has the significance stated in claim 1,

or

e) hydrolysing an enamine of general formula VIII

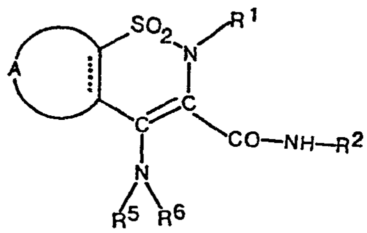

(VIII)

wherein A, $R^1$ and $R^2$ have the significance stated in claim 1, and $R^5$ and $R^6$ each signify lower

alkyl or form together with the nitrogen atom a pyrrolino, pyrrolidino, piperidino, morpholino or N-(lower alkyl)-piperazino group,

and

f) if desired, converting a thus-obtained compound of general formula I into a pharmaceutically acceptable salt.

7. Pharmaceutical preparation with anti-inflammatory, analgesic, anti-rheumatic and antithrombotic properties, containing a thienothiazine derivative of general formula I defined in claim 1 or a salt thereof and a carrier.

## Revendications

1. Dérivés de thiénothiazine de formule générale I:

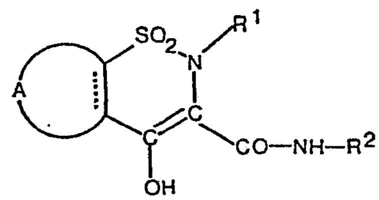

(I)

où A forme avec les deux atomes de carbone du noyau thiazine le groupe:

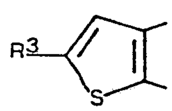 , 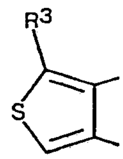 ou 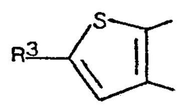

et où la ligne pointillée indique la double liaison présente dans le premier et dans le dernier cas, $R^1$ représente un alcoyle inférieur, $R^2$ représente un 2-thiazolyle, 4-méthyl-2-thiazolyle, 4,5-diméthyl-2-thiazolyle, 5-méthyl-1,3,4-thiadiazolyle, 2-pyrazinyle, 2-pyrimidinyle, 1,2,4-triazin-3-yle, 2-pyridyle, 3-pyridyle, 4-pyridyle, 3-méthyl-2-pyridyle, 4-méthyl-2-pyridyle, 5-méthyl-2-pyridyle, 6-méthyl-2-pyridyle, 4,6-diméthyl-2-pyridyle, 5-isoxazolyle, 5-méthyl-3-isoxazolyle, 3,4-diméthyl-5-isoxazolyle, 2,6-diméthyl-4-pyrimidinyle, 1,2,3,4-tétrazol-5-yle ou un radical phényle éventuellement substitué par un halogène, un hydroxy, un alcoyle inférieur, un trifluorométhyle ou un alcoxy inférieur et $R^3$ représente un halogène, ainsi que leurs sels.

2. Composé de formule générale I définie dans la revendication 1, où A représente le groupe:

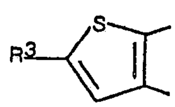

et où $R^3$ représente un halogène.

3. Composés selon la revendication 1, où $R^1$ représente un méthyle, $R^2$ un 2-pyridyle et $R^3$ un chlore ou un brome.

4. 6 - chloro - 4 - hydroxy - 2 - méthyl - 3 - (2 - pyridylcarbamoyl) - 2H - thiéno[3,2 - e]-1,2 - thiazin - 1,1 - dioxyde.

5. 6 - chloro - 4 - hydroxy - 2 - méthyl - 3 - (2 - pyridylcarbamoyl) - 2H - thiéno[2,3 - e]-1,2 - thiazin - 1,1 - dioxyde.

6. Procédé de préparation de dérivés de thiénothiazine de formule générale I définie dans la revendication 1 et de leurs sels, caractérisé en ce que:

a) on fait réagir un composé de formule générale II:

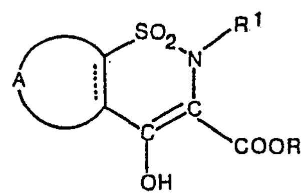

(II)

où R représente un alkyle inférieur et A et $R^1$ ont les significations indiquées dans la revendication 1, avec une amine de formule générale III:

$$H_2N—R^2 \qquad (III)$$

où $R^2$ a la signification indiquée dans la revendication 1, ou

b) on cyclise un dérivé fonctionnel réactif d'un acide de formule générale IV:

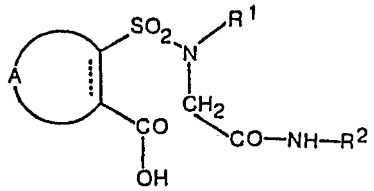

(IV)

où A, $R^1$ et $R^2$ ont les significations indiquées dans la revendication 1, ou

c) on alkyle un composé de formula générale V:

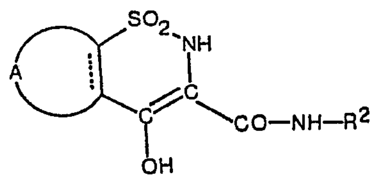

(V)

où A et $R^2$ ont les significations indiquées dans la revendication 1 avec un agent donnant le radical $R^1$ ou

d) on fait réagir un composé de formule générale VI:

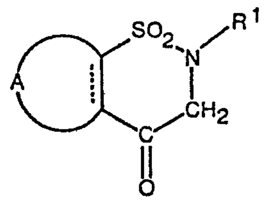

(VI)

où A et $R^1$ ont les significations indiquées dans la revendication 1, en présence d'une base forte, avec un isocyanate de formule générale VII:

$$O{=}C{=}N{-}R^2 \qquad \text{(VII)}$$

où $R^2$ a la signification indiquée ci-dessus, ou

e) on hydrolyse une énamine de formule générale VIII:

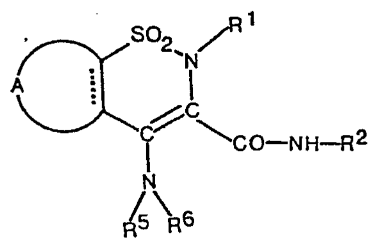

(VIII)

où A, $R^1$ et $R^2$ ont les significations indiquées dans la revendication 1 et $R^5$ et $R^6$ représentent chacun un alkyle inférieur ou forment, ensemble avec l'atome d'azote, un radical pyrrolino, pyrrolidino, pipéridino, morpholino ou N-(alkyle inférieur)-pipérazino, et

f) si on le désire, on transforme un composé de formule générale I ainsi obtenu en un sel pharmaceutiquement acceptable.

7. Préparation pharmaceutique à propriétés anti-inflammatoires, analgésiques, anti-rhumatismales et anti-thrombotiques, contenant un dérivé de thiénothiazine de formule générale I définie dans la revendication 1 ou un de ses sels et un véhicule.